Europäisches Patentamt

European Patent Office

Office européen des brevets

(19)

(11) Publication number: **0 213 898 B1**

## EUROPEAN PATENT SPECIFICATION

(12)

(45) Date of publication of patent specification: 19.06.91    (51) Int. Cl.5: **C12N 15/76**, C12N 1/20,
//(C12N1/20,C12R1:465)

(21) Application number: 86306508.2

(22) Date of filing: 21.08.86

(54) A host-vector system.

(30) Priority: 21.08.85 JP 181688/85

(43) Date of publication of application:
11.03.87 Bulletin 87/11

(45) Publication of the grant of the patent:
19.06.91 Bulletin 91/25

(84) Designated Contracting States:
AT BE CH DE FR GB IT LI LU NL SE

(56) References cited:

THE JOURNAL OF ANTIBIOTICS, vol. 36, no.
2, February 1983, pages 99-108; M. OKANISHI
et al.: "Basic techniques for DNA cloning
and conditions required for streptomycetes
as a host"

JOURNAL OF GENERAL MICROBIOLOGY, vol.
129, no. 9, September 1983, pages 2703-2714,
SGM, GB; E. KATZ et al.: "Cloning and ex-
pression of the tyrosinase gene from Strep-
tomyces antibioticus in Streptomyces
lividans"

BIOTECHNOLOGY, January 1984, pages
63-72; J.F. MARTIN et al.: "Cloning and ex-
pression of antibiotic production genes"

(73) Proprietor: SANKYO COMPANY LIMITED
No. 1-6, 3-chome Nihonbashi Honcho Chuo-
ku
Tokyo(JP)

(72) Inventor: Manome, Taichi c/o Sankyo Fermen-
tation Res. Lab.
Sankyo Company Limited 2-58, 1-chome,
Hiromachi
Shinagawa-ku Tokyo 140(JP)

(74) Representative: Gibson, Christian John Robert
et al
MARKS & CLERK 57/60 Lincoln's Inn Fields
London WC2A 3LS(GB)

EP 0 213 898 B1

**Description**

The present invention relates to host-vector systems.

DNA recombination has been particularly developed in micro-organisms such as Escherichia coli, Bacillus subtilis and yeast, especially Escherichia coil. The experiments on DNA recombination in Escherichia coli have reached the stage that they can be applied not only for analysis of various genes but also for the industrial production of some valuable peptides.

Meanwhile, actinomycete bacteria have for a long time been important in fermentation technology. Some, but not all, actinomycetes produce useful antibiotics and other physiologically active substances. However, the means for developing improved strains of actinomycetes are restricted. Within this restriction, some successes have been achieved, such as a higher productivity.

At present, host-vector systems are established in certain actinomycetes (for example, Streptomyces coelicolor A (3)2, Streptomyces lividans, etc), making it possible for some actinomycete genes to be cloned. Nevertheless, there is a need for further experimental systems for DNA recombination for use in research on breeding and improving actinomycetes. It is hoped to achieve higher productivity and production of new substances by these methods.

Thus, in producer micro-organisms of the family Actinomycetales, there is often a rate-limiting step which undesirably reduces the potential production of antibiotics or other useful substances. Mutation, recombinant plasmid formation, and other techniques of genetic engineering are methods which are generally applicable for developing improved or modified strains of such micro-organisms.

For example, with Streptomyces jumonjinensis, which is a producer of cephamycin C and related $\beta$-lactams, there is a rate-limiting enzyme in the biosynthetic pathway to cephamycin C. It might be hoped that the gene which codes this enzyme could be integrated into a recombinant plasmid and then transferred to the host in order to raise the productivity of cephamycin C. More generally, genes which code for one or more conversion enzymes might be integrated into recombinant plasmids and then transferred to actinomycete hosts in order to obtain good yields of desired products.

Plasmid pIJ702, described by Katz et al. in J. Gen. Microbiol. 129, 2703 (1983), is an artificially formed plasmid which can be employed to transform actinomycete bacteria. Streptomyces lividans transformed with plasmid pIJ702 has been deposited with the Japanese Fermentation Research Institute, FRI, and accorded the deposit number FERM-BP 1141. Samples of the micro-organism are readily available from the Fermentation Research Institute, and the plasmid pIJ702 can easily be isolated from the host Streptomyces lividans. Suitable isolation methods include that of Chater et al., Curr. Topics Microbiol. Immunol. 96 1159 (1982).

Plasmid pIJ702 contains the tyrosinase gene (the " mel" gene), which codes for melanin synthesis and thus for melanoid forms. When the tyrosinase gene is properly expressed, a black, diffusible pigment is produced and the culture medium becomes dark, for example from brown to deep black. Hence Mel+ forms can easily be detected and distinguished from Mel− or weakly Mel+ forms.

Furthermore, the plasmid pIJ702 contains the thiostrepton-resistance gene (the " tsr" gene), which codes for resistance against the antibiotic thiostrepton. When the thiostrepton-resistance gene is properly expressed, the bacterium is resistant to thiostrepton at a level for example of 50 $\mu$g/ml. Hence Thio r forms can easily be detected.

In fact, thiostrepton is of limited availability in Japan and elsewhere. However, transformed strains can also be detected by using an analogous antibiotic, thiopeptin, to which thiostrepton-resistant bacteria are cross-resistant. Thus, thiopeptin-resistance is synonymous with thiostrepton-resistance and is equally indicative of Thio r forms.

It will therefore be understood that the plasmid pIJ702 is useful for transformation of actinomycete hosts, in that it can impart two marker characteristics, melanism and thiostrepton resistance. Successful transformation of various species of Streptomyces with plasmid pIJ702 is described in the article by Katz et al. cited above.

In the tyrosinase gene in plasmid pIJ702, there are single insertional inactivation sites for three restriction enzymes, Sac I (Sst I), Bgl II and Sph I. By using these sites, the absence of a melanoid form can be taken as a selective marker for insertional inactivation in thiostrepton resistant strains.

The plasmid pIJ702 might thus be useful as a cloning vector, having the potential advantage that strains which contain recombinant plasmids with inserted DNA fragments can be easily detected and selected.

However, for many strains of actinomycetes, the potential of plasmid pIJ702 is not fulfilled.

For example, with some strains, transformation be achieved with plasmid pIJ702, but the resultant host-vector systems show only thiostrepton resistance, and do not exhibit melanism. The Katz et al. article, cited above, mentioned that transformation of Streptomyces clavuligerus with plasmid pIJ702 gives transformants

2

which are Mel⁻ or weakly Mel⁺. The lack of expression of the Mel⁺ phenotype in Streptomyces clavuligerus was not due to permanent alteration in the tyrosinase gene, since transformation of Streptomyces lividans with plasmid DNA recovered from the transformed Streptomyces clavuligerus yielded typical Mel⁺ transformants of plasmid pIJ702.

With other strains, it is often found that transformation is possible to give a transformed strain expressing thiostrepton resistance and melanism can be obtained, but that such strains are obtained only at low frequency.

Another handicap in development work with plasmid pIJ702 in actinomycetes is the frequent presence in host bacteria of other, naturally occuring plasmids. The other plasmids complicate the separation of recombinant plasmids for subsequent use as vectors, and also complicate the gene analysis.

In general, there remain problems with the use of plasmid pIJ702 in transformation of actinomycete bacteria.

An object of this invention is to provide improved recombinant plasmids derived from plasmid pIJ702. A specific object is to provide recombinant plasmids derived from plasmid pIJ702 which can be used to transform to a Mel⁺ phenotype a bacterium of the kind which is Mel⁻ or weakly Mel⁺ when transformed with plasmid pIJ702.

A further object resides in the detection of DNA sequences which have useful gene regulation activity and which can be inserted for instance in to the tyrosinase gene of plasmid pIJ702 to allow melanin synthesis in bacteria which do not give melanin synthesis when transformed with plasmid pIJ702.

More generally, the objects of this invention are concerned with improvements in host-vector sytems, where the host is an actinomycete bacterium and the vector is a derivative of plasmid pIJ702..

The present invention is based on experiments using plasmid p IJ702, where it has been found that there are some actinomycete bacteria which are incapable of expressing the tyrosinase gene of plasmid pIJ702. Such actinomycetes occur naturally or occur through mutation to remove any plasmids present in the bacterium.

In its broadest aspects, the invention provides a host-vector system. The host is a micro-organism of the order Actinomycetales, and the vector is a recombinant plasmid.

Specifically, the host micro-organism is one which before transformation has the characteristics that it is competent to transformation with plasmid pIJ702 and that when it is transformed with the plasmid pIJ702, the transformed micro-organism is able to express the thiostrepton-resistant gene of plasmid pIJ702 and is not able to express the tyrosinase gene of plasmid pIJ702. Such micro-organisms are thus Thio ʳ Mel⁻ - (where Mel⁻ includes weak Mel⁺) when transformed with plasmid pIJ702, which is contrary to the expectation of Thio ʳMel⁺.

The recombinant plasmid of this invention comprises plasmid pIJ702 and a DNA sequence having an expression-regulating function. The DNA sequence has an expression-regulating function in the sense that when the micro-organism is transformed with the recombinant plasmid, the transformed micro-organism is able to express both the thiostrepton-resistant gene of plasmid pIJ702 and the tyrosinase gene of plasmid pIJ702. Thus, the recombinant plasmid is one which gives the desired traits in the host of Thio ʳ Mel⁺.

As a result of the inclusion of the DNA sequence with an expression-regulating function, the tyrosinase gene is properly expressed in transformed hosts. In this way, melanism can be taken as a marker for succesful incorporation of the recombinant plasmid, and the absence of melanism can be taken as indicative of insertional inactivation.

Accordingly, this invention may be advantageously employed in developing and cloning an actinomycete host-vector system including foreign DNA, the characteristic of no melanism being used as marker for the foreign DNA. In this way, the production of a valuable peptide originally produced by another micro-organism might be possible by integrating into the recombinant plasmid the gene coding for the peptide.

There seems a good possibility that the peptide will be secreted in the medium through the protein-secreting system of actinomycetes.

Inclusion of the DNA fragment having an expression-regulating function can often potentiate production of the melanism pigment, allowing more immediate detection of successfully transformed hosts. It might also be expected that expression of any foreign gene contained in the plasmid is likewise potentiated by the effect of this DNA fragment.

The present invention is generally applicable to actinomycetes bacteria, that is, the family Actinomycetales, especially the streptomycetes bacteria of the genus Streptomyces such as Streptomyces jumonjinensis, Streptomyces clavuligerus or Streptomyces kasugaensis.

As a first step towards improved host-vector systems, a micro-organism freed of plasmids is obtained. Some micro-organisms of the order Actinomycetales do not contain interfering plasmids, and can be used

3

directly, while others do contain interfering plasmids and need to be rendered free of plasmids.

In the mutation method, the micro-organism before mutation has the characteristics (i) that it is competent to transformation with plasmid pIJ702 and (ii) that when it is transformed with the plasmid pIJ702, the transformed micro-organism is able to express the thiostrepton-resistant gene of pIJ702 and is able to express the tyrosinase gene of pIJ702. After mutation, for instance by chemical reagents or radiation, a micro-organism is selected which has the characteristics (i) that it is competent to transformation with plasmid pIJ702, and (ii) that when it is transformed with plasmid pIJ702, the transformed micro-organism is able to express the thiostrepton-resistant gene of pIJ702 and is not able to express the tyrosinase gene of pIJ702.

Examples of micro-organisms which need mutation include strains of Streptomyces jumonjinensis and Streptomyces kasugaensis. In general, the aim of the mutation is to free the micro-organism of plasmids, such as the two plasmids which normally occur in Streptomyces jumonjinensis, or the three plasmids normally present in Streptomyces kasugaensis.

To further consider the example of Streptomyces jumonjinensis SANK 66165 FERM-P 1545 NRRL 5741, it is a cephamycin and clavulanic acid-producing strain of actinomycetes which occurs with two plasmids, pSJ1 and pSJ2. The two plasmids have nearly the same molecular weight. Treatment with acriflavine and protoplast selection readily yields mutants free of the plasmids pSJ 1 and pSJ 2. One particular such mutant is Streptomyces jumonjinensis [16]-8.SANK 61185, described in more detail below.

The host free of plasmids is then ready for genetic engineering development work, specifically transformation with the improved recombinant plasmid. Suitable recombinant plasmids comprise plasmid pIJ702 containing an expression-regulating, SphI streptomycete-derived DNA fragment at the SphI site of the tyrosinase gene, capable of inducing expression of the tyrosinase gene.

Such SphI DNA fragments can be detected by the steps of:

inserting DNA fragments into the tyrosinase gene of plasmid pIJ702;

transforming a micro-organism which has the characteristics (i) that it is competent to transformation with plasmid pIJ702 and (ii) that when it is transformed with the plasmid pIJ702, the transformed micro-organism is able to express the thiostrepton-resistant gene of pIJ702 and is not able to express the tyrosinase gene of pIJ702; and

detecting any expression of the tyrosinase gene in the transformed micro-organism.

Thus, the recombinant plasmid can be obtained by a method of this invention which comprises

obtaining a micro-organism of the order Actinomycetales which micro-organism has the characteristics (i) that it is competent to transformation with plasmid pIJ702 and (ii) that when it is transformed with the plasmid pIJ702, the transformed micro-organism is able to express the thiostrepton-resistant gene of pIJ702 and is not able to express the tyrosinase gene of pIJ702;

transforming the micro-organism with a recombinant plasmid;

screening the transformed micro-organism for a clone expressing the tyrosinase gene;

culturing the clone expressing the tyrosinase gene; and

isolating the plasmid contained in the cultured clone.

The DNA fragment possessing a gene-regulating function is typically inserted at the Sph I site of the tyrosinase gene of plasmid pIJ702. Insertion can readily give a DNA sequence which can be used in construction of DNA for imparting tyrosinase gene expression capability to a micro-organism of the order Actinomycetales. In one modification of this invention, the DNA fragment is inserted in to the tyrosinase gene of plasmid pIJ702 after excision of the gene from the plasmid. For example, a Bcl I-cleaved fragment of plasmid pIJ702 can be used to provide the tyrosinase gene (there are two Bcl I sites in plasmid pIJ702 which are conveniently adjacent to but not within the tyrosinase gene). Such a modification of this invention finds further applications in genetic engineering of actinomycete bacteria.

For the recombinant plasmids of this invention, the DNA fragment possessing a gene-regulating function can be obtained for example from any of the streptomycetes. Suitable sources include but are not limited to the following strains, for which experimental results are given in the ensuing Examples:

Streptomyces venezuelae SANK 64477 JCM 4526 (JCM: Japan Collection of Micro-organisms);

Streptomyces fradiae SANK 69270 NRRL B-1195;

Streptomyces kanamyceticus SANK 61774 ATCC 21252;

Streptomyces niveus SANK 92870;

Streptomyces erythreus SANK 62279 NRRL 3885;

Streptomyces rimosus SANK 62171 ATCC 10970;

Streptomyces omiyaensis SANK 61277 NRRL B-1587;

Streptomyces griseus SANK 61784 IAM 0089 (IAM: Institute of Applied Microbiology, Tokyo University);

Streptomyces flavovirens SANK 61784;

Streptomyces sp. AN-351 SANK 61884;

Streptomyces jumonjinensis BS727 SANK 66782;

Streptomyces jumonjinensis AF-94 SANK 67882 FERM P-7081;

Streptomyces jumonjinensis[16]-8 SANK 61185; and

Streptomyces jumonjinensis[16]-9 SANK 67982.

In general, the total DNA is extracted from the chosen source, using for instance the method of J. Marmur (J. Mol. Biol. 3, 208 (1961) to give the donor DNA. The plasmid pIJ702 and the donor DNA can then be mixed in a suitable ratio, preferably from 1:2 to 1:8 of plasmid DNA to donor DNA, for example in the ratio of one part plasmid DNA to five parts of the donor DNA, and treated with a restriction enzyme. The restriction enzyme is preferably Sph I, though testing will establish if other candidate restriction enzymes give functional insertion. The treatment with the restriction enzyme typically involves incubation for from 1 to 5 hours at 25 to 40 °C, for example about 2 hours at around 37 °C. After cleavage, the restriction enzyme can be inactivated, for example by heating or treatment with an inactivating reagent such as phenol. The cleaved DNA can then be circularised, for example by the action of T4 DNA ligase for 10 to 20 hours at 10 to 20 °C, to give a mix of ligated DNA.

The actinomycete host typically as protoplasts can then be transformed with the DNA, and screened for successful host-vector products. For transformation, the host itself may be obtained by conventional culture methods and the cultured cells then washed and suspended in a hypertonic sucrose solution. The cell walls can then be lysed, for example with lysozyme, to give a protoplast preparation. The protoplasts can then be mixed with the ligated DNA mixture in the presence of a transformation aid such as polyethyleneglycol (PEG). After transformation followed by washing on a hypertonic medium, the protoplasts can be plated out and assayed. For example, the protoplasts can be spread on an agar plate and cultivated at 28 °C. After initial growth (say 10 to 30 hours), thiopeptin in a soft agar can be layered on to the agar cultures in order selectively to eliminate the colonies which are not transformed to thiopeptin resistance. Culturing is continued for instance at 28 °C, and the transformed strains can be discriminated from the parent strain by the presence or absence of thiopeptin resistance. Furthermore, the transformed strains containing the recombinant plasmid with a DNA fragment able to bring about the expression of the tyrosinase gene can be discriminated from other colonies by their brown or black color due to melanism.

## THE STRAIN S. JUMONJINENSIS [16]-8.SANK 61185

The morphological and physiological properties of this mutant strain [16]-8.SANK 61185 are given below in sections (1) to (5), along with deposit details at section (6) and a summary of the transformation experiments with this strain, sections (7) and (8). The preparation of the various agar media, the primary culture, the secondary culture and observations of the resultant growth were carried out according to the ISP standard, the Japanese Examination Standard for Inventions in Applied Microbiological Industry, recommendations by Waksman and the like criteria. Color tones on the culture media were taken from "Color Standard" (edited by Nihon Shikisai Kenkyasho).

### (1) Morphological characteristics

After culture of Streptomyces jumonjinensis [16]-8.SANK 61185 for 14 days at 28 °C, the substrate mycelia are in general found to be well branched and elongated lineally or curvilineally. Elongation in a zig-zag fashion and fragmentation of the substrate media are not observed. Formation of aerial mycelia is not observed on any of the tested agar or liquid media. Forms such as coremia or coremia-like growths and special organs such as sporangia or sclerotia are not observed.

### (2) Properties on various media

The culture characteristics were assessed on a variety of media at the 14th day of cultivation at 28 °C. In the following, G indicates growth, AM indicates aerial mycelium, R indicates reverse, and SP indicates soluble pigment.

| | | |
|---|---|---|
| Yeast-malt agar (ISP 2) | G: | Very good, rising or wrinkled, pale yellowish brown |
| | AM: | None |
| | R: | Pale yellowish brown |
| | SP: | None |

| | | |
|---|---|---|
| Oatmeal agar (ISP 3) | G: | Not so good, flat, pale yellowish orange |
| | AM: | None |
| | R: | Pale yellowish orange |
| | SP: | None |

Starch/          G:  Very good, wrinkled, pale
inorganic            yellowish brown
salt agar        AM: None
(ISP 4)          R:  Pale yellowish brown
                 SP: None

---

Glycerin/        G:  Not so good, flat, pale yellowish
asparagine           orange
agar             AM: None
(ISP 5)          R:  Pale yellowish orange
                 SP: None

---

Peptone          G:  Good, flat or wrinkled, pale
yeast-Fe             brown
agar             AM: None
(ISP 6)          R:  Pale yellowish brown
                 SP: None

---

Tyrosine         G:  Good, flat, pale brown
agar             AM: None
(ISP 7)          R:  Pale yellowish brown
                 SP: None

---

| Sucrose nitrate agar | G: | Not so good, flat, pale brown |
|---|---|---|
| | AM: | None |
| | R: | Pale brown |
| | SP: | None |

| Glucose/ asparagine agar | G: | Not so good, flat, pale yellowish brown |
|---|---|---|
| | AM: | None |
| | R: | Pale yellowish brown |
| | SP: | None |

| Nutrient Agar (Difco) | G: | Not so good, flat, pale brown |
|---|---|---|
| | AM: | None |
| | R: | Pale yellowish brown |
| | SP: | None |

| Potato/ carrot extract agar | G: | Good, flat, pale brown |
|---|---|---|
| | AM: | None |
| | R: | Pale brown |
| | SP: | None |

Water          G:   Not so good, flat, pale

agar               yellowish orange

               AM:  None

               R:   Pale yellowish orange

               SP:  None

---

(3) Physiological properties

The physiological properties are as follows:

hydrolysis of starch                          positive

liquefaction of gelatin                       positive

reduction of nitrate                          negative

coagulation of milk                           positive

peptonization of milk                         positive

temperature range for

    growth (Medium ISP 2)                     10 - 32°C

optimum temperature for

    growth (Medium ISP 2)                     25 - 30°C

salt resistance (Medium ISP 2)                >3% ~ <5%

substrate decomposing capacity

    casein                                    positive

    tyrosine                                  positive

    xanthine                                  negative

melanism (Medium ISP 1)                       negative

        (Medium ISP 6)                        negative

        (Medium ISP 7)                        negative

(4) Assimilation of carbon sources

Each carbon source in the table was added at 1% to a basal medium, Pridham-Gottlieb agar medium (ISP 9). The strain was inoculated on each medium and cultured at 28°C for 14 days. Assimilation was then assessed on the following scale:

```
++   well assimilable

 +   assimilable

 ±   poorly assimilable

 -   not assimilable.
```

```
D-glucose        +

L-arabinose      -

D-xylose         -

inositol         ++

D-mannitol       -

D-fructose       ±

L-rhamnose       -

sucrose          -

raffinose        -

control          -
```

(5) Cytochemical properties

As the main components of the cell wall, LL-DAP and glycine can be detected. Meso-DAP and 3-OH-DAP can not be found. This data shows that the cell wall of this strain is type I.

The mutant strain [16]-8.SANK 61185 exhibits microbiological properties substantially similar to those of its parent strain SANK 66165, except that the mutant forms far less aerial hyphae than the parent strain.

(6) Deposit

The mutant strain [16]-8-SANK 61185 was deposited at the Fermentation Research Institute on 17 august 1985 with the deposit number FERM BP-1140.

(7) Transformation with plasmid pIJ702

When the mutant strain [16]-8-SANK 61185 is employed as host for plasmid pIJ702, the frequency of transformed strains is about 100 times higher than the frequency for the parent strain SANK 66165. However, the transformed strain thus obtained expressed only thiopeptin resistance, but not melanism. The plasmid pIJ702 in the transformed strain [16]-8-SANK 61185 was separated out again, and the resulting plasmid was treated with various restriction enzymes. The cleavage pattern was then examined by agarose gel electrophoresis. The experimental results showed no difference between the re-separated plasmid and the original plasmid pIJ702. When the re-separated plasmid pIJ702 was employed to transform the original strain SANK 66165, the transformed strain expressed both thiopeptin resistance and melanism.

This finding reveals clearly that the lack of melanoid forms in the transformed strain is not to be attributed to any deficiency of the transferred plasmid pIJ702. The lack of melanism has to be attributed to another cause in the strain [16]-8-SANK 61185, such as mutation of RNA polymerase, which results in the inability to express the tyrosinase gene contained in plasmid pIJ702.

(8) Transformation with improved recombinant plasmids

In the present invention, a DNA fragment from DNA of another actinomycete micro-organism is able to compensate for the deficiency in the expression of the tyrosinase gene in the plasmid pIJ702 by the strain [16]-8-SANK 61185. In this way, cloning with the use of an absence of melanism as a marker for insertional inactivation becomes possible with this strain, and a valuable host-vector system wherein the host is strain [16]-8-SANK 61185 can be established.

Thus, when plasmid pIJ702 was cleaved with Sph I and DNA fragments obtained from Streptomyces venezuelae SANK 64477 were inserted, 3000 transformed thiopeptin-resistant strains were obtained, of which 17 colonies were forming melanoid pigments. Similar positive results were obtained with other sources for the DNA fragments, using DNA from 10 species (13 strains) of Streptomycetes. Only Sph I-cleaved fragments could induce the expression of the tyrosinase gene. Suitable fragments were obtained at a rate as high as 0.5% to 2%, and varied in size from 120 bp to 9000 bp. These results suggest that suitable DNA fragments are distributed widely within streptomycetes.

Strain [16]-8-.SANK 61185 was transformed again by transfer of the recombinant plasmids. The transformed strains obtained by re-transformation were Mel⁺ strains without exception. On the other hand, transformed strains from strain [16]-8.SANK 61185 transformed as a control with original plasmid pIJ702 showed only thiopeptin resistance and were not Mel⁺.

The appearance of Mel⁺ strains with the recombinant plasmids indicates that the tyrosinase gene remains intact and that the expression of this structure gene can be induced by the action of the inserted DNA fragment.

In summary, for transformation of strain [16]-.8 SANK 61185, an Sph I-cleaved DNA fragment of Streptomyces origin can induce the expression of tyrosinase gene contained in plasmid pIJ702 in the following circumstances:

(1) It can be successfully cloned when cleavage is effected with Sph I, for which the DNA sequence at the recognition site is GCATG↑C. Desired clones appear with a relatively high frequency, compared in general with the isolation rate for any one gene.

(2) The size of the DNA fragments capable of inducing the expression of the tyrosinase gene is not fixed. Even small fragments considered too small to code the structure gene for melanistic forms can induce melanism.

(3) Induction of the expression of tyrosinase gene requires proper orientation of the inserted DNA fragment.

From these three points, it may be deduced that the Sph I-cleaved DNA fragment of chromosomal DNA origin does not contain the structure gene for melanism, but contains a DNA sequence recognizable by the strain [16]-8.SANK 61185. Such a DNA sequence has an expression-regulating function, so that the tyrosinase gene in original plasmid pIJ702 is expressed. An example of a DNA sequence having an expression-regulating function and requiring a proper orientation is a promoter.

From the foregoing, it will be understand that the host-vector system provided by this invention is potentially of great value. A strain containing a new recombinant plasmid with an inserted foreign DNA fragment can be easily distinguished by formation or not of melanoid colonies, that is by so-called insertional inactivation. Furthermore, an examination of the strength of the expression of the tyrosinase gene may give intelligence on the strength of expression induced by the DNA sequence with the expression-regulating function. It can be expected that a desired valuable peptide can be expressed by ligating, downstream from this DNA fragment, a gene that codes for this peptide in place of the tyrosinase gene. Thus, the method of this invention for detecting DNA fragments possessing a gene-regulating function is likely to be very useful in a general way.

The present invention is illustrated by the following non-limiting Examples. In the Examples, reference is made to the accompanying drawings:

Figure 1 illustrates a typical improved recombinant plasmid pMEL formed in Example 3 from plasmid pIJ702, and capable of inducing melanism in a host, strain [16]-8.SANK 61185.

Figure 2 illustrates recombinant plasmids pMEL16 and pSLF16-1 discussed in Example 5(1).
Figure 3 illustrates recombinant plasmids pMEL2 and pSLF2-20 discussed in Example 5(2).
Figure 4 illustrates recombinant plasmids pMEL22 and pSLF22-11 discussed in Example 5(3).

Example 1: Culturing of Streptomyces jumonjinensis, strain [16]-8.SANK 61185 and transformation of this strain using plasmid pIJ702

Spores were collected from a stock culture of Streptomyces jumonjinensis strain SANK 66165, inoculated on a fresh YM agar plate (YM agar: glucose 0.4%, malt extract 1%, yeast extract 0.4%, agar 2%; pH 7.2), and incubated for 10 to 14 days at $28°C$ to allow good formation of spores. The spores were scraped off and suspended in 5 ml of sterile distilled water. The suspension was filtered with a glass filter (type 3G3), and the filtrate passed through a filter paper (Whatmann No 2) to give a spore suspension lacking link-form spores. The spore suspension was spread on a YM agar plate containing acriflavine at 0.2 $\mu m/ml$, and incubated at $28°C$ to allow growth of colonies.

The growth colonies were transferred on a YM agar slant medium, and the colonies used to inoculate a $GGC^Y$ liquid medium (glycerol 0.4%, glycine 0.1%, casamino acid 0.4%, magnesium chloride 0.1%, calcium chloride 0.01%, yeast extract 0.1%, and trace salts solution 4 ml/l). Shake culturing was then effected at $28°C$ for 3 days. The microbial cells were collected and suspended in a TES buffer solution (tris-(hydroxymethyl)-aminomethane (tris) 50 mM, EDTA 50 mM, sodium chloride 50 mM, pH 7.5) and lysed with lysozyme. The lysed solution was centrifuged at 15,000 rpm for 60 minutes to obtain the supernatant.

Plasmid was generally detectable in this lysed supernatant by agarose gel electrophoresis, though the plasmids pSJ 1 and pSJ 2 of this strain SANK 66165 are few in number, 1 to 2 for each bacterial cell. The lysed supernatant was treated with RNase and then with Pronase (trade mark), and polyethyleneglycol was added to a final concentration of 10%. This mixture was allowed to stand overnight at $4°C$ to precipitate DNA.

After concentration, the presence or absence of plasmids was examined by agarose gel electrophoresis. The strain of Streptomyces jumonjinensis thus obtained, designated strain AF-94, was shown to have only plasmid pSJ 2, and no plasmid pSJ 1.

In order to free the strain AF-94 of plasmid pSJ 2, a protoplast preparation of this strain was made and returned to a hypertonic condition. Growing colonies were cultured in GGC $^Y$ liquid medium. A strain designated [16]-8.SANK 61185 was observed to be free from both plasmid pSJ 1 and pSJ 2. The lack of plasmid DNA in this strain was also confirmed by labelling of DNA with $^3H$-thymidine followed by fractionation using cesium choride/ethidium bromide equilibrium density-gradient centrifugation.

Transformation of strain [16]-8.SANK 61185 by plasmid pIJ702 was achieved according to a modified method of Bibb et al. (Nature 274, 398 (1980)). Mycelia of strain [16]-8.SANK 61185 were inoculated in 20 ml of GGC $^Y$ medium in a 50-ml distillation flask, and incubated at $24°C$ to $28°C$ for 72 hours on a reciprocal shaker at 120 rpm. The growth obtained was inoculated to a level of 5% in 100 ml of GGC $^Y$ medium in a 500-ml Sakaguchi flask, and incubated at $24°$ to $28°C$ for 24 hours on a reciprocal shaker. From this culture solution, a mycelial pellet was obtained by low speed centrifugation.

The mycelial pellet was suspended in 20 ml of medium P (medium P: sucrose 320 mM. TES buffer solution 25 mM, sodium chloride 70 mM, MgCl2 10 mM and CaCl2 20 mM), washed and centrifuged to give a cell pellet which was then suspended again in 20 ml medium P. To this suspension, 1 ml of a 40 mg/ml lysozyme solution was added, and protoplasts of strain [16]-8.SANK 61185 were formed after incubation for 1 hour at $28°C$. A mixture of protoplasts and mycelia which had not been lyzed was filtered off with a glass filter (type 3G3) to leave a solution rich in protoplasts. The protoplast solution was then centrifuged at low speed and the resulting pellet was suspended again in medium P. This process was repeated three times, and by sufficient washing, a protoplast solution of sufficient strength was prepared.

The protoplast solution was centrifuged to give a pellet. The transformation process was as a rule then carried out at room temperature. 100 $\mu l$ of medium P containing plasmid pIJ 702 was mixed with the pellet, and the mixture stirred slowly in order homogeneously to disperse the protoplasts. To the homogenate was added 0.5 ml of medium P containing 20% polyethyleneglycol, and the mix allowed to stand for 1 minute. A further 4 ml of medium P was then added. After transformation, a protoplast pellet was obtained by centrifugation. The pellet was mixed with 5 ml of medium P, stirred and adequately washed, followed by centrifuging. This process was repeated at least three times.

The transformed protoplasts were suspended in a suitable amount of medium P and then spread on an agar plate of medium R2MP prepared particularly for growth of melanistic forms (R2MP medium: sucrose 120 g, K2SO4 0.25 g, K2HPO4 0.05 g, MgCl2.6H2O 10.12 g. CaCl2.2H2O 2.95 g, glucose 4 g, casamino acid 0.1 g, L-proline 3 g, DL-norleucine 0.05 g, tyrosine 0.5 g, yeast extract 2 g, malt extract 5 g, TES buffer soution (pH 7.2) 100 ml, trace salts solution 2 ml, and agar 20 g, in 1000 ml). The spreading on the $R_2MP$ agar plate was followed by incubation at $28°C$ for 20 hours. 3 ml of soft agar $R_3$ medium ($R_3$ medium: sucrose 120 g, $K_2HPO_4$ 0.2 g, $MgCl_2.6H_2O$ 8.1 g, $CaCl_2.2H_2O$ 2.2 g, 250 mM TES buffer solution (pH 7.2) 100 ml, glucose 10 g, yeast extract 4 g, polypeptone 4 g, KCl 0.5 g and agar 5 g in 1000 ml) containing thiopeptin at 50 $\mu$g/ml was layered on the $R_2MP$ agar plate. Incubation of the plate was then continued at $28°C$.

Transformed strains resistant to thiopeptin were observed to grow, but their frequency was only $10^{-3}$ to $10^{-2}$. None of these thiopeptin resistant strains showed melanism.

No difference was observed in the cleavage pattern with various restriction enzymes respectively between the plasmid re-isolated from the transformed strain and the original plasmid pIJ702. When the re-isolated plasmid was transferred into the original strain SANK 66165, the strain thus transformed showed melanism as well as thiopeptin resistance.

Example 2: Cloning of DNA fragments of various streptomycetes origins to give expression in strain [16]-8.SANK 61185 of the tyrosinase gene contained in plasmid pIJ702

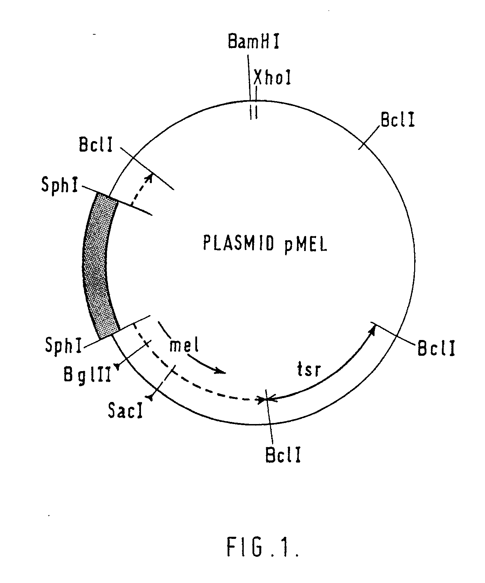

As shown in Example 1, the tyrosinase gene in plasmid pIJ702 is not expressed in strain [16]-8.SANK 61185. Accordingly, DNA fragments capable of achieving the expression of the tyrosinase gene were inserted and cloned. To this end, suitable DNA fragments were extracted from the mycelia of various streptomycetes bacteria (11 species from 14 strains), according to Marmur's method. The resultant plasmids form the pMEL series of plasmids, for which the construction is generally shown in Figure 1.

(1) To one part of a mixture of 5 $\mu$g of DNA from Streptomyces venezuelae SANK 64477 and 1 $\mu$g of plasmid pIJ702 was added one-third part by volume of a fourfold diluted buffer solution suited for restriction enzyme reaction, and restriction enzyme Sph I 9 $^{\text{u}}$. For complete cleavage of DNA, the mixture was incubated at a $37°C$ for 2 hours. The mixture was then heated at $70°C$ for 10 minutes to inactivate the restriction enzyme. one-tenth part by volume of 3M sodium acetate solution was added to one part of the reaction mixture and the mixture stirred. The mixture was then added to 2.5 parts by volume of ethanol cooled previously to $-20°C$, and the resultant mix cooled at $-70°C$ for 10 to 20 minutes. The solution was centrifuged using a microcentrifuge and the supernatant discarded.

The resultant DNA precipitate was washed with ethanol cooled previously to $-20°C$, dried in vacuo and dissolved in sterile distilled water. To one part of this solution was added one-ninth part in volume of a tenfold diluted buffer solution suited for DNA ligase reaction (buffer solution: Tris HCl 660 mM, $MgCl_2$ 66mM, DTT 100 mM, ATP 1.1 mM, pH 7.6), and $T_4$ DNA ligase. After incubation of the resultant solution for 16 hours at $14°C$, the ligase was inactivated by heating for 10 minutes at $65°C$. By using the preparation thus obtained, transformation of strain [16]-8.SANK 61185 was carried out in a similar manner to that in Example 1. 3000 transformed strains resistant to thiopepton were obtained, of which 17 strains, 0.6% of 3000, were observed to be melanoid ($Mel^+$).

Transformation using a preparation cleaved respectively with Bgl II or Sac I was carried out in a similar manner several times, but no melanoid forms were observed among the transformed strains.

The 17 $Mel^+$ strains were incubated in GGC $^Y$ medium and cell pellets obtained. The cell pellets were suspended in TES buffer solution and lysed with lysozyme. After addition of 10% sarcosine for completing the lysis, the suspension was centrifuged (15,000 rpm, 60 min) to give a lysed supernatant which was then subjected to gel electrophoresis on 0.8% agarose. From the electrophoretic mobility it was concluded that the 17 $Mel^+$ strains contained, without exception, recombinant plasmids with inserted DNA fragments. As a control experiment, 16 strains were randomly selected from among the transformed strains showing only thiopeptin resistance, and, after lysis, their plasmids were examined in a similar manner. It was found that 11 strains contained the plasmid pIJ702 and 5 strains contained recombinant plasmids with inserted DNA fragments.

This experimental result shows that insertion of a DNA fragment into the Sph I-cleaved site does not always bring about $Mel^+$ formation.

(2) DNA of 10 Streptomyces species other than Streptomyces venezuelae was used in a similar manner to Example 2(1) to obtain transformed $Mel^+$ strains. The transformed $Mel^+$ strains capable of melanism as well as possessing thiopeptin resistance, were obtained from every strain at a frequency of 0.5 to 2% but only when the DNA was cleaved with Sph I. The $Mel^+$ strains were incubated in $GGC^Y$ medium, lysed and examined for plasmids. The plasmids were all shown to be recombinant plasmids.

Example 3: Isolation of the recombinant plasmid from $Mel^+$ strains and determination of the size of the inserted DNA fragments

The recombinant plasmids from Mel+ strains of transformed strain [16]-8.SANK 61185 were isolated. Mycelia of each Mel+ strain was inoculated in 20 ml of a growth medium (growth medium: glucose 0.4%, malt extract 1.0%, and yeast extract 0.4%) in a 50-ml distillation flask, and incubated on a reciprocal shaker for about 72 hours at 24° to 28°C. The suspension was added at 1 to 5% of the amount of medium to 100 ml of a second growth medium (growth medium: glycerol 0.4%, casamino acid 0.4%, yeast extract 0.05%, malt extract 0.1%, $MgSO_4$ 0.1%, $CaCO_3.2H_2O$ 0.01%, $KH_2PO_4$ 0.2%, and $Na_2HPO_4.12H_2O$ 0.8%) in a 500-ml Sakaguchi flask, and the inoculated medium was incubated on a reciprocal shaker for 24 to 48 hours at 24° to 28°C.

Mycelia were collected from the incubated solution by low speed centrifugation (for instance 10,000 × g, 4°C. 20 minutes) and a mycelial pellet obtained after removal of the supernatant by decantation. The mycelial pellet was suspended again in 20 ml of TES buffer solution (25 mM tris-(hydroxymethyl)-aminomethane (Tris), 25 mM EDTA and 25 mM sodium choride, pH 7.5). Then, 1 ml of 40 mg/ml lysozyme solution was added to the suspension. The mixture was kept at 37°C under slow stirring for 5 to 15 minutes, then 3 ml of 10% sodium dodecyl sulfate (SDS) solution was added. After careful mixing, the mixed solution was kept at 37°C for 5 minutes to allow lysis.

The lysed solution was centrifuged at 40,000 × g, at 4°C for 30 minutes to obtain a crude lysate as the supernatant. To one part of the lysate was added a quarter part of 5 M saline solution to give a final concentration of sodium chloride of 1 M. The saline mix was cooled at 0°C for 2 to 3 hours to precipitate the SDS added previously. After removal of SDS by centrifugation at 3,000 × g, at 0°C for 15 minutes, ribonuclease was added to the supernatant and the mixture incubated for 20 minutes at 37°C. Pronase was added and incubation continued for 20 minutes at 37°C. 40% polyethyleneglycol (PEG) solution was added to the resultant solution to a final concentration of 10%.

The mixture was allowed to stand overnight at 0°C in order to precipitate DNA. After discarding the supernatant from low speed centrifugation (3,000 × g, at 0°C for 15 minutes), the precipitate was suspended in 4.7 ml of TES buffer solution until thoroughly dissolved. The solution was dialyzed in TES buffer solution and an extracted DNA sample was obtained.

The extracted DNA sample was mixed with cesium choride and then with ethidium bromide (EtBr), a fluorescence developing reagent, to prepare a solution of density 1.620. The solution was subjected to equilibrium density gradient centrifugation at 150,000 × g, at 18°C for 40 hours. When the centrifugal tube was irradiated with UV at 320 nm, covalently closed circular DNA was observed in the tube as a separately formed fluorescent band, under a strong fluorescent band due to linear DNA of chromosomal origin.

The fluorescent band due to ccc DNA was isolated, and one part was extracted with one part of n-butyl alcohol three times to remove ethidium bromide. The water layer was dialyzed against a suitable buffer solution (such as 10 mM Tris, 10 mM sodium choride and 1 mM EDTA, pH 7.5) to obtain a purified recombinant plasmid preparation. The concentration of the purified recombinant plasmid was determined by the UV absorbancy at 260 nm.

The size of the DNA fragment inserted into the recombinant plasmid was determined by agarose gel electrophoresis. By cleaving 0.5 μg of the recombinant plasmid with restriction enzyme Sph I, two bands were formed. One band was due to a linearized fragment of pIJ702, employed as the vector plasmid, and the other band was due to the inserted DNA fragment. A Hind III-cleaved fragment of λ DNA and a Hae III-cleaved fragment of φ χ174 DNA were employed for molecular weight markers. The size of the inserted DNA fragment was determined by mobility of the marker in electrophoresis when the concentration of agarose gel was 0.8%, 1.2% or 2%, depending on the fragment size. The sizes of the inserted DNA fragments are shown in the following table along with data for the number of Mel+ plasmids in proportion to the total number of Thio+ strains, and the pMEL designations for the Mel+ plasmids.

## TABLE

| strain | Mel$^+$ | size (bp) | plasmids |
|---|---|---|---|
| S. venezuelae | | | |
| SANK 64477 | 17/3000 | 240-4100 | pMEL1-17 |
| S. sp. AN-351 | | | |
| SANK 61884 | 7/300 | 120-1540 | pMEL18-24 |
| S. flavovirens | | | |
| SANK 61784 | 3/450 | 120-? | pMEL25-27 |
| S. jumonjinensis Bs727 | | | |
| SANK 66782 | 5/440 | | |
| S. jumonjinensis AF-94 | | | |
| SANK 67882 | 3/278 | 240-9000 | pMEL101-119 |
| S. jumonjinensis [16]-8 | | | |
| SANK 61185 | 9/882 | | |
| S. jumonjinensis [16]-9 | | | |
| SANK 67982 | 2/183 | | |
| S. fradiae | | | |
| SANK 69270 | 3/576 | 280-560 | pMEL201-203 |
| S. kanamyceticus | | | |
| SANK 61774 | 1/145 | 260 | pMEL301 |
| S. niveus | | | |
| SANK 92870 | 1/207 | 130 | pMEL401 |
| S. erythreus | | | |
| SANK 62279 | 1/254 | 620 | pMEL501 |
| S. rimosus | | | |
| SANK 62171 | 2/236 | 490-600 | pMEL601-602 |
| S. omiyaensis | | | |
| SANK 61277 | 2/241 | 590-? | pMEL701-702 |
| S. griseus | | | |
| SANK 61784 | 1/135 | 1600 | pMEL801 |

Example 4: Re-transformation of strain [16]-8.SANK 61185 by transfer of the recombinant plasmid obtained from Mel$^+$ strains

Strain [16]-8.SANK 61185 was re-transformed by transfer of the purified recombinant plasmid preparation obtained in Example 3. The re-transformation was carried out according to the procedure of Example 1. The transformed strains from the re-transformation were, without exception, thiopeptin resistant and were Mel$^+$ strains capable of melanism. Thus, the inserted DNA fragments contained a DNA sequence capable of inducing the expression of the tyrosinase gene in the strain [16]-8.SANK 61185. This suggests that, although the DNA fragments inserted into the re-combinant plasmids were indefinite in size, they each have a DNA sequence capable of inducing the expression in strain [16]-8.SANK 61185 of the tyrosinase gene contained in Plasmid pIJ702.

Example 5: The relation between the orientation of the DNA fragment inserted into the recombinant plasmids of the Mel+ strains and the expression of the tyrosinase gene

From Example 3, the Table, three kinds of recombinant plasmids are illustrative, namely pMEL16, pMEL2 and pMEL22.

(1) Isolation of a plasmid into which the DNA fragment inserted in the recombinant plasmid pMEL16 is inversely inserted

The size of the DNA fragment inserted into the recombinant plasmid pMEL16 is about 240 bp. One μg of this plasmid was cleaved with the restriction enzyme Sph I. The enzyme was inactivated by heating for 10 minutes at 70°C. T4 DNA ligase was employed for DNA recombination. By transfer of the recombinant plasmid preparation, strain [16]-8.SANK 61185 was transformed according to the procedure in Example 1. A number of non-melanoid strains (Mel−) were observed to grow, as well as Mel+ strains.

32 strains were randomly selected from the Mel− strains in order to examine the plasmids by agarose gel electrophoresis according to the procedure given in Example 2. Three strains, including strain SLF 16-1, were found to have a plasmid with the same mobility as that of original recombinant plasmid pMEL16. From the strain SLF 16-1, a purified plasmid pSLF16-1 was isolated according to the procedure described in Example 3. The plasmid pSLF16-1 was cleaved with restriction enzyme Sph I and the resultant DNA fragment was compared by agarose gel elecrophoresis with the Sph I-cleaved fragment of the original plasmid pMEL16. It was confirmed that plasmid pSLF16-1 possessed the same 240 bp inserted DNA fragment as in the original plasmid pMEL16. When this plasmid pSLF16-1 was transferred to strain [16]-8.SANK 61185, the resulting transformed strains which grew were all Mel− strains, showing only thiopeptin resistance.

The fact that plasmid pSLF16-1 with the same inserted DNA fragment as in the original recombinant plasmid pMEL 16 did not induce the expression of the tyrosinase gene might suggest that the DNA fragment inserted into the plasmid pSLF16-1, is in inverse orientation to that in the recombinant plasmid pMEL16, accounting for the failure to express the tyrosinase gene.

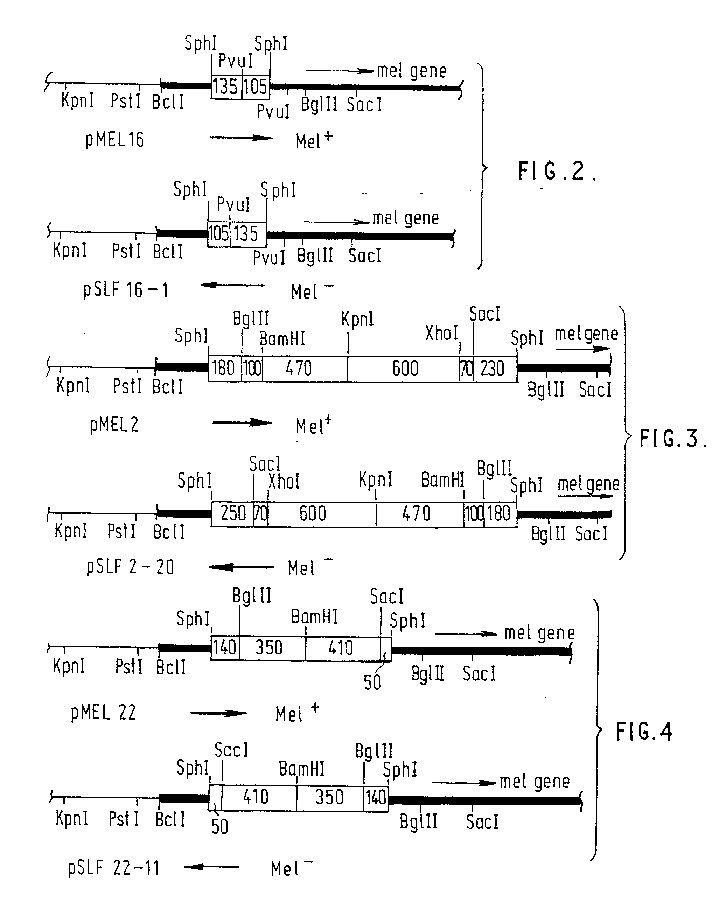

This inversion is shown in Figure 2 and was also confirmed by an experiment where plasmids pSLF 16-1 and pMEL16 were doubly cleaved with restriction enzyme Sac I and Pst I, each resulting in a DNA fragment of 1040 bp which was cleaved with restriction enzyme Pvu I. The sizes of the DNA fragments thus obtained were determined by polyacrylamide gel electrophoresis.

(2) Isolation of a plasmid into which the DNA fragment inserted in the recombinant plasmid pMEL2 is inversely inserted

The DNA fragment inserted into the recombinant plasmid pMEL2 is as large as about 1650 bp. The DNA fragment has sites cleavable with 5 kinds of restriction enzyme, Bgl II, Bam HI, Kpn I, Xho I and Sac I. These restriction enzymes are moreover each known to cleave plasmid pIJ702 at one site. Therefore, the orientation can be determined easily by comparing the sizes of DNA formed after cleavage using these restriction enzymes. Isolation of a plasmid in which the DNA fragment inserted into plasmid PMEL2 is inversely inserted was carried out in a similar manner to that for obtaining the plasmid PMEL16. A strain with plasmid pSLF2-20 was detected which was equal in size to pMEL2 and not capable of inducing the expression of the tyrosinase gene.

A purified preparation of plasmid pSLF2-20 was isolated and employed for transformation of strain [16]-8.SANK 61185. Only Mel− strains were obtained. Both plasmids pSLF2-20 and pMEL2 were cleaved with the 5 restriction enzymes mentioned above and the sizes of the resultant DNA fragments were determined by gel electrophoresis on 1.2% agarose. The electrophoretic pattern demonstrated that the DNA fragment was inserted into plasmid pSLF2-20 in inverse orientation to that in the original recombinant plasmid pMEL2, as shown in Figure 3.

(3) Isolation of a plasmid into which the DNA fragment inserted in the recombinant plasmid pMEL22 is inversely inserted

The DNA fragment inserted into the recombinant plasmid pMEL22 is about 950 bp in size and has sites cleavable with 3 kinds of restriction enzymes, Bgl II, Bam HI and Sac I. These enzymes are also known each to cleave plasmid pIJ702 at one site. Therefore, the orientation can be determined easily in a manner similar to Example 5(2).

Isolation of a plasmid into which the DNA fragment inserted into plasmid pMEL22 is inversely inserted was carried out in a similar manner to that for obtaining pMEL16 or pMEL2, and plasmid pSLF22-11 was obtained which was equal in size to pMEL22 and not capable of inducing the expression of the tyrosinase gene. Both plasmids pSLF22-11 and pMEL22 were cleaved with the 3 restriction enzymes, Bgl II, Bam HI and Sac I, and the sizes of resultant DNA fragments were determined by 2.0% agarose gel electrophoresis. The electrophoretic pattern showed that the DNA fragment was inserted into plasmid pSLF22-11 in inverse orientation to that inserted into the original recombinant plasmid pMEL22, as shown in Figure 4.

## Claims

1. A micro-organism of the order Actinomycetales when transformed with a recombinant plasmid,
   the micro-organism before transformation having the characteristics that it is competent to transformation with plasmid pIJ702 and that when it is transformed with the plasmid pIJ702, the transformed micro-organism is able to express the thiostrepton-resistant gene of pIJ702 and is not able, or only weakly able, to express the tyrosinase gene of pIJ702;
   the said recombinant plasmid comprising plasmid pIJ702 containing an expression-regulating, SphI streptomycete-derived sequence at the SphI site of the tyrosinase gene, enabling the said micro-organism, when transformed with the recombinant plasmid, to express both the thiostrepton-resistant gene of pIJ702 and the tyrosinase gene of pIJ702.

2. A micro-organism of the order Actinomycetales when transformed with a recombinant plasmid,
   the micro-organism before transformation being free of plasmids;
   the said recombinant plasmid comprising plasmid pIJ702 containing an expression-regulating, SphI streptomycete-derived sequence at the SphI site of the tyrosinase gene, enabling the said micro-organism, when transformed with the recombinant plasmid, to express both the thiostrepton-resistant gene of pIJ702 and the tyrosinase gene of pIJ702.

3. A transformed micro-organism according to claim 1 or 2, wherein the micro-organism is of the species Streptomyces jumonjinensis, Streptomyces clavuligerus or Streptomyces kasugaensis.

4. A method of producing a recombinant plasmid, which method comprises
   obtaining a micro-organism of the order Actinomycetales which micro-organism has the characteristics (i) that it is competent to transformation with plasmid pIJ702 and (ii) that when it is transformed with the plasmid pIJ702, the transformed micro-organism is able to express the thiostrepton-resistant gene of pIJ702 and is not able, or only weakly able, to express the tyrosinase gene of pIJ702;
   transforming the micro-organism with a recombinant plasmid comprising plasmid pIJ702 which contains an expression-regulating, SphI streptomycete-derived sequence at the SphI site of the tyrosinase gene capable of inducing expression of the tyrosinase gene;
   screening the transformed micro-organism for a clone expressing the tyrosinase gene;
   culturing the clone expressing the tyrosinase gene; and
   isolating the plasmid contained in the cultured clone.

5. A recombinant plasmid comprising plasmid pIJ702 containing an expression-regulating, SphI streptomycete-derived DNA fragment at the SphI site of the tyrosinase gene, enabling expression of the mel$^+$ phenotype; the recombinant plasmid being of use for transforming a competent micro-organism of the order Actinomycetales, said micro-organism before transformation having the characteristic that when it is transformed with plasmid pIJ702, the transformed micro-organism is able to express the thiostrepton-resistant gene of pIJ702 and is not able, or only weakly able, to express the tyrosinase gene of pIJ702; and the transforming of the micro-organism with the recombinant plasmid giving a clone with the characteristic that it is able to express both the thiostrepton-resistant gene of pIJ702 and the tyrosinase gene of pIJ702.

6. A method of detecting a DNA fragment as defined in Claim 5, the method comprising the steps of:
   inserting a DNA fragment into the tyrosinase gene of plasmid pIJ702 at the SphI site;

transforming a micro-organism which has the characteristics (i) that it is competent to transformation with plasmid pIJ702 and (ii) that when it is transformed with the plasmid pIJ702, the transformed micro-organism is able to express the thiostrepton-resistant gene of pIJ702 and is not able, or only weakly able, to express the tyrosinase gene of pIJ702; and

detecting any expression of the tyrosinase gene in the transformed micro-organism.

7. A DNA fragment as defined in Claim 5.

8. A DNA sequence which can be used in construction of DNA for imparting tyrosinase gene expression capability to a micro-organism of the order <u>Actinomycetales</u>, the DNA sequence comprising a DNA fragment with gene-regulating function, the DNA fragment being as defined in claim 5, and at least the tyrosinase gene of plasmid pIJ702, the DNA fragment being inserted at the Sph I site of the tyrosinase gene of plasmid pIJ702.

## Revendications

1. Un micro-organisme de l'ordre des <u>Actinomycétales</u> lorsqu'il est transformé avec un plasmide recombinant,

le micro-organisme avant transformation ayant comme caractéristiques qu'il peut être transformé avec le plasmide pIJ702 et que, lorsqu' il est transformé avec le plasmide pIJ702, le micro-organisme transformé est capable d'exprimer le gène de résistance au thiostrepton de pIJ702 et n'est pas capable ou est seulement faiblement capable d'exprimer le gène de la tyrosinase de pIJ702;

ledit plasmide recombinant comprenant le plasmide pIJ702 contenant une séquence SphI dérivée de streptomycète régulatrice d'expression au site SphI du gène de la tyrosinase permettant au micro-organisme, lorsqu' il est transformé avec le plasmide recombinant, d'exprimer à la fois le gène de la résistance au thiostrepton de pIJ702 et le gène de la tyrosinase de pIJ702.

2. Un micro-organisme de l'ordre des <u>Actinomycétales</u> lorsqu' il est transformé avec un plasmide recombinant,

le micro-organisme avant transformation étant libre de plasmides;

ledit plasmide recombinant comprenant le plasmide pIJ702 contenant une séquence SphI régulatrice d'expression dérivée de streptomycète au site SphI du gène de la tyrosinase, permettant audit micro-organisme, lorsqu' il est transformé avec le plasmide recombinant, d'exprimer à la fois le gène de résistance au thiostrepton de pIJ702 et le gène de la tyrosinase de pIJ702.

3. Un micro-organisme transformé suivant la revendication 1 ou 2 dans lequel le micro-organisme est de l'espèce <u>Streptomyces</u> <u>jumonjinensis</u>, <u>Streptomyces</u> <u>clavuligerus</u> ou <u>Streptomyces</u> <u>kasugaensis</u>.

4. Une méthode de production d'un plasmide recombinant, laquelle méthode comprend

l'obtention d'un micro-organisme de l'ordre des <u>Actinomycétales</u>, lequel micro-organisme a les caractéristiques (i) qu'il peut être transformé avec le plasmide pIJ702 et (ii) que, lorsqu' il est transformé avec le plasmide pIJ702, le micro-organisme transformé est capable d'exprimer le gène de la résistance au thiostrepton de pIJ702 et n'est pas capable ou est seulement faiblement capable d'exprimer le gène de la tyrosinase de pIJ702;

la transformation du micro-organisme avec un plasmide recombinant comprenant le plasmide pIJ702 qui contient une séquence SphI dérivée de streptomycète régulatrice d'expression au site SphI du gène de la tyrosinase capable d'induire l'expression du gène de la tyrosinase;

le criblage du micro-organisme transformé pour obtenir un clone exprimant le gène de la tyrosinase;

la culture du clone exprimant le gène de la tyrosinase et

l'isolement du plasmide contenu dans le clone cultivé.

5. Un plasmide recombinant comprenant le plasmide pIJ702 contenant un fragment d'ADN SphI dérivé de streptomycète régulateur d'expression au site SphI du gène de la tyrosinase permettant l'expression du phénotype mel$^{+}$; le plasmide recombinant étant utile pour transformer un micro-organisme compétent de l'ordre des <u>Actinomycétales</u>, ledit micro-organisme ayant, avant tranformation, la caractéristique que, lorsqu' il est transformé avec le plasmide pIJ702, le micro-organisme transformé est capable d'exprimer le gène de la résistance au thiostrepton de pIJ702 et n'est pas capable ou seulement faiblement capable d'exprimer le gène de la tyrosinase de pIJ702, et la transformation du micro-organisme avec le plasmide recombinant pour donner un clone avec la caractéristique qu'il est capable d'exprimer à la fois le gène de la résistance au thiostrepton de pIJ702 et le gène de la tyrosinase de pIJ702.

6. Une méthode de détection d'un fragment d'ADN tel que défini dans la revendication 5, la méthode comprenant les stades de:

insertion d'un fragment d'ADN dans le gène de la tyrosinase du plasmide pIJ702 au site SphI;

transformation d'un micro-organisme qui a les caractéristiques (i) qu'il peut être transformé avec le plasmide pIJ702 et (ii) que, lorsqu' il est transformé avec le plasmide pIJ702, le micro-organisme transformé est capable d'exprimer le gène de la résistance au thiostrepton de pIJ702 et n'est pas capable ou seulement faiblement capable d'exprimer le gène de la tyrosinase de pIJ702 et

la détection de toute expression du gène de la tyrosinase dans le micro-organisme transformé.

7. Un fragment d'ADN tel que revendiqué dans la revendication 5.

8. Une séquence d'ADN qui peut être utilisée dans la construction d'ADN pour donner la capacité d'expression du gène de la tyrosinase à .un micro-organisme de l'ordre des Actinomycétales, la séquence d'ADN comprenant un fragment d'ADN avec la fonction de régulation de gène, le fragment d'ADN étant tel que défini dans la revendication 5 et au moins le gène de la tyrosinase du plasmide pIJ702, le fragment d'ADN étant inséré au site SphI du gène de la tyrosinase du plasmide pIJ702.

## Ansprüche

1. Ein mit einem Rekombinationsplasmid transformierter Mikroorganismus der Gattung Actinomycetales, wobei

der Mikroorganismus vor der Transformation für eine Transformation durch das Plasmid pIJ702 zugänglich ist und der durch das Plasmid pIJ702 transformierte Mikroorganismus in der Lage ist das thiostreptonresistente Gen von pIJ702 zu exprimieren und nicht befähigt ist oder nur in geringem Maße befähigt ist das Tyrosinasegen von pIJ702 zu exprimieren und

das Plasmid pIJ702 aufweisende Rekombinationsplasmid an der SphI-Stelle des Tyrosinasegens eine expressionsregelnde, von Streptomyceten abgeleitete SphI-Sequenz enthält, welche die durch das Rekombinationsplasmid transformierten Mikroorganismen befähigt sowohl das thiostreptonresistente Gen von pIJ702 als auch das Tyrosinasegen von pIJ702 zu exprimieren.

2. Ein durch ein Rekombinationsplasmid transformierter Mikroorganismus der Gattung Actinomycetales, wobei

der Mikroorganismus vor der Transformation frei von Plasmiden ist und

das Plasmid pIJ702 aufweisende Rekombinationsplasmid an der SphI-Stelle des Tyrosinasegens eine expressionsregelnde, von Streptomyceten abgeleitete SphI-Sequenz enthält, welche die durch das Rekombinationsplasmid transformierten Mikroorganismen befähigt sowohl das thiostreptonresistente Gen von pIJ702 als auch das Tyrosinasegen von pIJ702 zu exprimieren.

3. Transformierter Mikroorganismus nach Anspruch 1 oder 2, worin der Mikroorganismus zu den Spezien Streptomyces jumonjinensis, Streptomyces clavuligerus oder Streptomyces kasugaensis gehört.

4. Verfahren zum Herstellen eines Rekombinationsplasmids, bei welchem

ein zur Gattung Actinomycetales gehörender Mikroorganismus beigestellt wird, welcher die Eigenschaft besitzt (i) für eine Transformation durch Plasmid pIJ702 zugänglich zu sein und (ii) im durch das Plasmid pIJ702 transformierten Zustand in der Lage ist das thiostreptonresistente Gen von pIJ702 zu exprimieren und nicht befähigt ist oder nur in geringem Maße befähigt ist das Tyrosinasegen von pIJ702 zu exprimieren,

der Mikroorganismus mit einem Rekombinationsplasmid transformiert wird, welches ein Plasmid pIJ702 aufweist, das an der SphI-Stelle des Tyrosinasegens eine expressionsregelnde, von Streptomyceten abgeleitete SphI-Sequenz enthält, die ein Exprimieren des Tyrosinasegens bewirkt,

der transformierte Mikroorganismus auf ein das Tyrosinasegen exprimierendes Klon gesichtet wird,

das das Tyrosinasegen exprimierende Klon kultiviert wird und

das im kultivierten Klon enthaltene Plasmid isoliert wird.

5. Ein Rekombinationsplasmid, welches ein Plasmid pIJ702 aufweist, das an der SphI-Stelle des Tyrosinasegens ein expressionsregelndes, von Streptomyceten abgeleitetes SphI-DNS-Fragment enthält, welches das Exprimieren des Mel[+]--Phänotyps ermöglicht, wobei das Rekombinationsplasmid für das Transformieren eines zugänglichen Mikroorganismus der Gattung Actinomycetales verwendbar ist, dieser Mikroorganismus vor der Transformation die Eigenschaft besitzt, daß er sobald er durch das Plasmid pIJ702 transformiert ist, der transformierte Mikroorganismus in der Lage ist das thiostreptonresistente Gen von pIJ702 zu exprimieren und nicht befähigt ist oder nur in geringem Maße befähigt ist das Tyrosinasegen von pIJ702 zu exprimieren, und das Transformieren des Mikroorganismus mit dem Rekombinationsplasmid ein Klon ergibt, welches befähigt ist sowohl das thiostreptonresistente Gen von pIJ702 als auch das Tyrosinasegen von pIJ702 zu exprimieren.

6. Verfahren zum Feststellen eines in Anspruch 5 definierten DNS-Fragments, bei welchem Verfahren
ein DNS-Fragment in das Tyrosinasegen des Plasmids pIJ702 an der SphI-Stelle eingeführt wird,
ein Mikroorganismus transformiert wird, welcher die Eigenschaft besitzt (i) für eine Transformation durch das Plasmid pIJ702 zugänglich zu sein und (ii) im durch das Plasmid pIJ702 transformierten Zustand in der Lage zu sein das thiostreptonresistente Gen von pIJ702 zu exprimieren und nicht befähigt zu sein oder nur in geringem Maße befähigt zu sein das Tyrosinasegen von pIJ702 zu exprimieren, und
jedwede Exprimierung des Tyrosinasegens im transformierten Mikroorganismus festgestellt wird.

7. DNS-Fragment der in Anspruch 5 definierten Art.

8. DNS-Sequenz, welche dazu verwendet werden kann DNS aufzubauen, die einem Mikroorganismus der Gattung Actinomacetales die Fähigkeit vermittelt Tyrosinasegen zu exprimieren, wobei die DNS-Sequenz ein in Anspruch 5 definiertes DNS-Fragment mit genregelnder Funktion und zumindest das Tyrosinasegen des Plasmids pIJ702 enthält und das DNS-Fragment an der SphI-Stelle des Tyrosinasegens des Plasmids pIJ702 eingeführt ist.

20

FIG.1.

FIG.2.

FIG.3.

FIG.4